# EUROPEAN PATENT SPECIFICATION

(11) **EP 2 696 690 B1**
(45) Date of publication and mention of the grant of the patent: **04.10.2017**
(21) Application number: 12770690.1
(22) Date of filing: 16.04.2012
(51) Int. Cl.: A61K 31/095, A61B 1/04

(54) **SULFATE SALTS AS TRANSIT TIME ENHANCER**
SULFATSALZE ZUR VERBESSERUNG DER TRANSITZEIT
SELS DE SULFATE EN TANT QU'AGENT AMÉLIORANT LE TEMPS DE TRANSIT

(30) Priority: 15.04.2011 US 201161475938 P
(43) Date of publication of application: 19.02.2014
(73) Proprietor: Braintree Laboratories, Inc., Braintree, MA 02185-0929 (US)
(72) Inventor: PELHAM, Russell, W., Duxbury MA 02332 (US); GAT, Daniel, Zichron Yaakov 30900 (IL); ROSENTHAL, Shirrie, Zrufa 30850 (IL)
(74) Representative: Grund, Martin
(86) International application number: PCT/US2012/033769
(87) International publication number: WO 2012/142572

(56) References cited:
- US-A1- 2003 202 957
- Xu Wei-Wei Jiang Ning Fan A Macro Lubbing: "OMOM Capsule Endoscopy Nursing - Research Papers Center", Clinical Medicine Papers, 23 September 2009 (2009-09-23), pages 1-5, XP055141501, Retrieved from the Internet: URL:http://eng.hi138.com/medicine-papers/c linical-medicine-papers/200909/145815_omom -capsule-endoscopy-nursing.asp#.VBv9clPsx8 E [retrieved on 2014-09-19]
- DIPALMA ET AL.: 'A Randomized Clinical Study Evaluating the Safety and Efficacy of a New, Reduced-Volume, Oral Sulfate Colon-Cleansing Preparation for Colonoscopy' AM J GASTROENTEROL vol. 104, 2009, pages 2275 - 2284, XP055092478
- WI ET AL.: 'Bowel Preparation for Capsule Endoscopy: A Prospective Randomized Multicenter Study.' GUT AND LIVER vol. 3, no. 3, September 2009, pages 180 - 185, XP055092475
- BRAINTREE, MA.: 'SUPREP Bowel Prep Kit - Prescribing Information and Medication Guide' August 2010, XP055106834 Retrieved from the Internet: <URL:http://www.braintreelabs.com/Collatera l/Documents/English-US/SUPREP%20PI-Med%208- 10.pdf>
- OCCHIPINTI ET AL.: 'How to choose the best preparation for colonoscopy' NAT. REV. GASTROENTEROL. HEPATOL. vol. 6, 2009, pages 279 - 286, XP008168181
- MELMED ET AL.: 'Capsule Endoscopy: Practical Applications' CLINICAL GASTROENTEROLOGY AND HEPATOLOGY vol. 3, 2005, pages 411 - 422, XP005120749

## Description

### CROSS REFERENCE TO RELATED APPLICATIONS

This application claims the benefit of U.S. Provisional Patent Application Ser. No. 61/475,938, entitled "Sulfate Salts as Transit Time Enhancer," which was filed April 15, 2011.

### FIELD OF THE INVENTION

This disclosure is in the field of medicine, and more particularly, relates to endoscopy and colonoscopy of the bowels.

### BACKGROUND

Gastrointestinal (GI) disease of the intestines present in many forms. For example, GI cancers, such as colorectal cancer and cancers of the small intestine, are a major cause of morbidity in the Western world. Polyps and bleeding from the small intestine or colon are among the hallmarks of these GI cancers. Bleeding from the small intestine or colon may also be caused by Crohn's disease, ulcers, drug-induced small bowel injury, and benign tumors.

It is often difficult to diagnose which of these disorders is the cause of GI symptoms such as pain or bleeding. In addition to the disorders described above, 30% to 40% of bleeding from the small bowel is due to abnormal blood vessels that lie within the bowel wall. These arteriovenous malformations (AVMs) are associated with a number of serious disorders not related to the bowel, including chronic kidney disease, and valvular heart disease. Thus, as bleeding from the GI tract may be indicative of a life-threatening disorder, its speedy and accurate diagnosis should be addressed.

Capsule endoscopy has become a first-line tool to evaluate these GI disorders and to detect AVMs, especially when traditional endoscopy and enteroscopy fail to do so. Capsule endoscopy entails the use of a capsular endoscope the size of a large pill (a "capsule endoscope") containing a strong light source, a transmitter, an antenna, and a camera, all powered by a battery. Once swallowed, it moves via peristalsis and/or mechanical propulsion through the esophagus, stomach, and small intestine while transmitting multiple pictures per second to a receiver for the patient. The capsule is then eliminated through the colon. Within about five to eight hours after ingestion, the patient returns the receiver to the doctor, who downloads the information onto a computer and reviews in detail the video images and other data, looking for a disorder or abnormalities, for example, those that are the source of the pain or bleeding.

In some cases, the capsule endoscope does not view the entire small bowel or colon, either because it has become stuck in the small intestine, or because the battery has run out prior to completing its trip through the GI tract. In other cases, it is desirable to speed up capsule endoscope transit so as to provide diagnostic information more rapidly than would otherwise be expected. Having a means to control or speed up transit through, and wetting, the GI tract would therefore be advantageous. Accordingly, the ability to control and increase the propulsive movements of the bowels should enhance the results obtained from capsule endoscopy.

Before capsule endoscopy is commenced, it is important that the GI tract of the patient be prepared for the procedure. This includes cleansing the small bowel of chyme and digestive fluids and the large bowel of stool, and filling them with a solution that aids the capsule endoscope in traveling quickly and easily through the small bowel and colon before the capsule endoscope battery runs out, and without obstructing transmittal of the pictures, yet providing high visual resolution.

Various solutions containing sodium phosphate (NaP) have been used to prepare patients for colon capsule endoscopy (see, *e.g*., Spada et al. (2011) J. Clin. Gastroenterol., Feb:45(2):119-24). Xu Wei-Wei Jiang Ning Fan A Macro Lubbing, "OMOM Capsule Endoscopy Nursing - Research Papers Center", Clinical Medicine Papers, 23. September 2009, pages 1-5, concerns capsule endoscopy and mentions that, for bowel preparation, the day before swallowing the capsule, the patients received a 50% magnesium sulfate solution for purging the gastrointestinal tract. Wi et al. (2009) "Bowel Preparation for Capsule Endoscopy: a Prospective Randomized Multicenter Study.", Gut and Liver 3(3):180-185, is a review on capsule endoscopy in which different bowel preparation methods are tested.

However, what is needed are improved solutions for preparing patients for capsule endoscopy which provide excellent cleansing, rapid stomach emptying, and rapid small bowel transit such that the entire gastrointestinal tract, including the small bowel and colon, is visualized.

### SUMMARY

The invention is defined by the appended claims. Accordingly, the invention provides the use of an oral sulfate solution to prepare the gastrointestinal tract of a patient for examining the interior of the gastrointestinal tract of the patient via capsule endoscopy, wherein the oral sulfate solution is to be administered to the patient in an amount effective to purge the gastrointestinal tract of the patient of its contents and to cleanse the gastrointestinal tract, and wherein the oral sulfate solution is a transit speed enhancer, comprises sodium sulfate, potassium sulfate, and magnesium sulfate, comprises about 0.0096 g/ml to about 0.50 g/ml sulfate in the form of an inorganic sulfate salt, and does not contain a phosphate salt. Moreover, the invention provides the use of an oral sulfate solution to prepare the gastrointestinal tract of a patient for determining the source, type, location, and cause of a gastrointestinal pathology in the patient, wherein the oral sulfate solution is to be administered to the patient experiencing the pathology to prepare the gastrointestinal tract of the patient for capsule colonoscopy, wherein the oral sulfate solution is to be administered in an amount effective to purge the gastrointestinal tract and to cleanse the gastrointestinal tract, and wherein the solution comprises magnesium sulfate, potassium sulfate and sodium sulfate, comprises about 0.0096 g/ml to 0.50 g/ml sulfate in the form of an inorganic sulfate salt, and does not contain a phosphate salt. Further, disclosed is a bowel preparation procedure for capsule endoscopy for the examination of the gastrointestinal tract. The procedure provides purging and excellent cleansing, rapid stomach emptying, and rapid small bowel (SB) transit. Also disclosed is an improved method of examining the interior of the small bowel and colon by capsule endoscopy. In addition, a method of identifying the source of a GI pathology is disclosed using an oral sulfate composition that enhances GI tract endoscopic procedures, including reducing the transit time of the capsule endoscope, and which purges and cleanses the bowels.

More specifically, a method of examining the interior of the gastrointestinal tract of a patient via capsule endoscopy is disclosed. The method comprises: administering an oral sulfate composition to the patient in an amount effective to purge the gastrointestinal tract of the patient of its contents and to cleanse the gastrointestinal tract, the oral sulfate composition being a transit speed enhancer and comprising an sulfate in the form of an inorganic sulfate salt and not containing a phosphate salt; orally administering an activated capsule endoscope to the patient; positioning a receiver to detect data transmitted from the activated capsule endoscope as it passes through the interior of gastrointestinal tract of the patient; and analyzing the data detected by the receiver before and/or after the administered capsule endoscope has been expelled from the colon of the patient.

The oral sulfate composition is a solution. In certain embodiments, the oral sulfate solution comprises about 0.0096 g/ml to 0.50 g/ml sulfate. In a particular embodiment, the oral sulfate solution comprises about 0.028 g/ml sulfate.

The solution comprises magnesium sulfate, potassium sulfate and sodium sulfate. In specific embodiments, the oral sulfate solution comprises about 0.0095 g/ml to , about 0.038 g/ml sodium, about 0.002 g/ml to about 0.009 g/ml potassium, about 0.0005 g/ml to about 0.05 g/ml magnesium, and about 0.02 g/ml to about 0.1 g/ml sulfate. In a particular embodiment, the oral sulfate solution comprises about 0.022 g/ml sodium, about 0.005 g/ml potassium, about 0.001 g/ml magnesium, and about 0.07 g/ml sulfate.

In some embodiments, about 15 ml to 1000 ml of the oral sulfate solution is to be administered. In certain embodiments, about 15 ml, about 20 ml, 25 ml, 30 ml, 35 ml, 40 ml, 45 ml, 50 ml, 55 ml, 60 ml, 65 ml, 70 ml, 75 ml, 80 ml, 85 ml, 90 ml, 95 ml, 100 ml, 150 ml, 200 ml, 250 ml, 300 ml, 350 ml, 400 ml, 450 ml, 500 ml, 550 ml, 600 ml, 650 ml, 700 ml, 750 ml, 800 ml, 850 ml, 900 ml, 950 ml, or 1000 ml of the sulfate solution is administered. In some embodiments, 15 ml of the oral sulfate solution is to be administered.

The oral sulfate solution is to be administered before, with, or after the administration of the capsule endoscope. In some embodiments, the oral sulfate solution is to be administered before and with the administration of the capsule endoscope. In another embodiment, the oral sulfate solution is to be administered before and after the administration of the capsule endoscope. In yet another embodiment, the oral sulfate solution is to be administered before, during, and after the administration of the capsule endoscope. In some embodiments, the oral sulfate solution is to be administered more than one time before or after the administration of the capsule endoscope. In certain embodiments, an osmotic laxative or a stimulant laxative is to be ingested before or after the administration of the oral sulfate solution and the capsule endoscope. In some embodiments, the oral sulfate solution is to be administered with, before, and/or after the capsule endoscope. In certain embodiments, the oral sulfate solution is to be administered more than one time before, during, and/or after the administration of the capsule endoscope.

In some embodiments, the receiver is placed proximal to the patient. In other embodiments, the receiver is placed on or under the patient.

In some embodiments, the gastrointestinal pathology is a hemorrhage, ulcer, polyp, lesion, precancerous lesion, cancer, diverticulitis, or inflammatory disorders including Crohn's disease, colitis, or ulcerative colitis.

In another aspect, disclosed is a method of determining the type, location, and cause of a gastrointestinal pathology in a patient. This method comprises: administering an oral sulfate composition to the patient experiencing the pathology to prepare the gastrointestinal tract of the patient for capsule colonoscopy, the oral sulfate composition, purging the gastrointestinal tract of the patient of its contents and cleansing the gastrointestinal tract, the composition comprising sulfate in the form of an inorganic sulfate salt, and not containing a phosphate salt; orally administering an activated capsule endoscope to the patient; positioning a receiver to detect and store photographic and other data transmitted from the capsule endoscope as it passes through the intestinal tract of the patient; and analyzing the data detected by the receiver before and/or after the administered capsule endoscope has been expelled from the colon of the patient, the data indicating the type, source and cause of the pathology.

The oral sulfate composition is a solution, such as one comprising about 0.0096 g/ml to 0.50 g/ml sulfate. In one embodiment, oral sulfate solution comprises about 0.028 g/ml sulfate. The solution comprises magnesium sulfate, potassium sulfate and sodium sulfate. In particular embodiments, the oral sulfate solution comprises about 0.0095 g/ml to about 0.038 g/ml sodium, about 0.002 g/ml to about 0.009 g/ml potassium, about .0005 g/ml to about 0.05 g/ml magnesium, and about 0.02 g/ml to about 0.1 g/ml sulfate. In a certain embodiment, the oral sulfate solution comprises about 0.022 g/ml sodium, about 0.005 g/ml potassium, about 0.001 g/ml magnesium, and about 0.07 g/ml sulfate.

In some embodiments, about 15 ml to 1000 ml of the oral sulfate solution is to be administered. In certain embodiments, about 15 ml, about 20 ml, 25 ml, 30 ml, 35 ml, 40 ml, 45 ml, 50 ml, 55 ml, 60 ml, 65 ml, 70 ml, 75 ml, 80 ml, 85 ml, 90 ml, 95 ml, 100 ml, 150 ml, 200 ml, 250 ml, 300 ml, 350 ml, 400 ml, 450 ml, 500 ml, 550 ml, 600 ml, 650 ml, 700 ml, 750 ml, 800 ml, 850 ml, 900 ml, 950 ml, or 1000 ml of the sulfate solution is administered.

In some embodiments, the oral sulfate solution is to be administered with, before, and/or after the capsule endoscope. In certain embodiments, the oral sulfate solution is to be administered more than one time before, during, and/or after the administration of the capsule endoscope.

The method described further comprises administering an osmotic laxative and/or a stimulant laxative before, during or after the administration of the oral sulfate composition and the capsule endoscope.

In some embodiments, the receiver is placed proximal to the patient. In certain embodiments, the receiver is placed on or under the patient, such as when the patient is reclining.

In some embodiments, the gastrointestinal pathology identified by the disclosed method is a hemorrhage, ulcer, polyp, lesion, precancerous lesion, cancer, diverticulitis, or inflammatory disorders including Crohn's disease, colitis, or ulcerative colitis.

### DESCRIPTION

The invention is defined by the appended claims. Patent and scientific literature referred to herein establishes knowledge that is available to those of skill in the art.

### Definitions

Unless otherwise defined, all technical and scientific terms used herein have the same meaning as commonly understood by one of ordinary skill in the art to which this invention belongs.

The articles "a" and "an" are used herein to refer to one or to more than one (*i.e.,* to at least one) of the grammatical object of the article. By way of example, "an element" means one element or more than one element.

The term "or" is used herein to mean, and is used interchangeably with, the term "and/or," unless context clearly indicates otherwise.

The term "about" is used herein to mean a value - or + 20% of a given numerical value. Thus, about 60% means a value of between 60% - 20% of 60 and 60% + 20% of 60 (*i.e.,* between 48% and 72%).

The term "composition" refers to a sulfate-containing pharmaceutical material or product which can be in the form of a solution, suspension, tablet, capsule, or powder.

The phrase "effective amount" as used herein means that amount of one or more agent, material, or composition comprising one or more agents according to the present disclosure that is effective for producing some desired effect in an animal. It is recognized that when an agent is being used to achieve a therapeutic effect, the actual dose which comprises the "effective amount" will vary depending on a number of conditions including, but not limited to, the physical form of the composition being administered (such as a solution or tablet), the particular condition being treated, the severity of the disease, the size and health of the patient, and the route of administration. A skilled medical practitioner can readily determine the appropriate dose using methods well known in the medical arts (see, *e*.*g*., Remington: The Science and Practice of Pharmacy (20th ed.) Limmer, Editor, Lipincott, Williams, & Wilkins (2000)).

In one nonlimiting example, an "effective amount" pertains to an amount of a sulfate composition which, after administration or ingestion, at least induces purgation of the GI tract of its contents. An effective amount also refers to an amount of a sulfate composition which ultimately (by way of purgation) results in cleansing of the GI tract.

The term "cleansing" is used herein to refer to removal of stool material from the bowel such that the bowel can be effectively examined, *e.g.,* by capsule endoscopy, colonoscopy or sigmoidoscopy. Cleansing can be the result of more than one procedure which causes purgation of the GI tract.

The term "purgation" is used herein to refer to evacuation of a copious amount of stool from the bowels after administration of a purgative dose of laxative.

The phrase "pharmaceutically acceptable" is employed herein to refer to those compounds, materials, compositions, and/or dosage forms which are, within the scope of sound medical judgment, suitable for use in contact with the tissues of human beings, animals, and plants without excessive toxicity, irritation, allergic response, or other problem or complication, commensurate with a reasonable benefit/risk ratio.

A "transit speed enhancer" or "transit booster" refers to the sulfate composition of the present disclosure which speeds up the transit time of the capsule endoscope or the time it takes for an ingested capsule endoscope to move through the GI tract of the patient.

A "patient" is a mammal, such as a human, cow, dog, cat, horse, elephant, etc.

The terms "administrating" and ingesting" are used interchangeably herein and refer to the oral provisional of something to the gastrointestinal tract of a patient by the patient or by a medical professional.

### Methods of Bowel Examination and GI Pathology Analysis

It has been discovered that an inorganic oral sulfate composition surprisingly and unexpectedly increases small bowel transit speed, and does so faster than do solutions containing sodium phosphate (NaP). This is the case even at the same or lesser doses of inorganic sulfate. The dose of inorganic sulfate which produces this effect is much lower than those known to stimulate gastric transit. These discoveries have been exploited to develop the present disclosure, which is directed, at least in part, to methods of examining the interior of the GI tract, and methods of identifying the source and cause of a GI pathology.

The method of the present disclosure, including a method of examining the interior of the GI tract, small bowel and/or colon, of a patient, is performed by orally administering to the patient an amount of an oral sulfate composition effective to purge and to cleanse the GI tract of the patient, and then orally administering an activated capsule endoscope to the patient. An "activated capsule endoscope" is one that is set to collect and transmit data. Such data can be photographic images, temperature reading, and/or pH reading, of the GI tract as the capsule is propelled through it. The capsule endoscope is activated according to a preset schedule or remotely by the physician or health care provider, for example.

The oral sulfate composition is administered before or contemporaneously with ingestion of the activated capsule endoscope, and may also be administered after capsule ingestion, such as one hour, two hours, three hours, four hours, or five hours after capsule ingestion.

After administration of the capsule endoscope, a receiver is used to detect the data in, or transmitted from, the activated capsule endoscope. As the capsule endoscope travels to the GI tract, it transmits data to the receiver, which is later analyzed to examine the interior of the GI tract, such as the small bowel and/or colon or to identify a GI pathology and/or its source.

The receiver is placed in any position which allows it to obtain or receive data from the capsule endoscope as it moves through the GI tract of the patient. The receiver can be placed on the patient, or under the patient, for example, when the patient is reclining, or it can be proximal to, but not touching, the patient. For example, the patient may be fitted with a receiver on the outside of her body.

A stimulant laxative, such as bisacodyl, senna, or picosulfate, may then be administered, e.g., as a suppository, foam, or aerosol, to aid in dispelling the capsule from the patient's body. The data from the receiver can then be used to determine the type, location, and cause of the pathology.

GI pathologies that can be identified by this method include, but are not limited to, lesions, precancerous lesions, ulcers, hemorrhages, cancer, polyps, diverticulitis, and inflammatory disorders such as Crohn's disease, colitis, and ulcerative colitis. The method also provides data which indicates the location of the pathology.

Before the administration of the oral sulfate composition and the activated capsule endoscope, the patient may be pretreated by the administration of a stimulant laxative, e.g., senna (about 25 mg to 150 mg), bisacodyl (about 5 mg to 20 mg), or picosulfate (about 5 mg to 20 mg), followed by a clear liquid diet. The patient may alternatively or additionally be pretreated with a osmotic laxative such as polyethylene glycol (PEG) solution (about 1 liter to about 4 liters), such as one containing PEG 3350, alone or with added electrolytes in water (such as in GoLYTELY^{®} or HalfLytely^{®}). Other useful osmotic laxatives include, without limitation, sorbitol, lactitol, lactulose, or Milk of Magnesia. The patient may alternatively or additionally be pretreated with an oral sulfate solution, such as one that comprises magnesium sulfate, sodium sulfate, or potassium sulfate in water. For example, a stimulant laxative (such as senna) may be administered two days before the capsule endoscopic procedure, and/or an osmotic laxative (such as PEG) may be administered on the evening before, the morning before, or the evening and morning before the procedure.

### Oral Sulfate Compositions

The oral sulfate composition of the present disclosure may be in the form of a solution (OSS) which contains at least two inorganic sulfate salts in a pharmaceutically acceptable carrier, such as water. The amount of OSS administered may be from about 15 ml to about 1000 ml.

In some embodiments, about 15 ml to 1000 ml of the oral sulfate solution is administered. In certain embodiments, about 15 ml, about 20 ml, about 25 ml, about 30 ml, about 35 ml, about 40 ml, about 45 ml, about 50 ml, about 55 ml, about 60 ml, about 65 ml, about 70 ml, about 75 ml, about 80 ml, about 85 ml, about 90 ml, about 95 ml, about 100 ml, about 150 ml, about 200 ml, about 250 ml, about 300 ml, about 350 ml, about 400 ml, about 450 ml, about 500 ml, about 550 ml, about 600 ml, about 650 ml, about 700 ml, about 750 ml, about 800 ml, about 850 ml, about 900 ml, about 950 ml, or about 1000 ml of the sulfate solution is administered.

The total amount of sulfate in the OSS is about .0096 g/ml to about 0.05 g/ml of solution, from about 0.05 g/ml to about 0.10 g/ml of solution, from about 0.6 g/ml to about 0.9 g/ml of solution, or from about 0.7 g/ml to about 0.8 g/ml of solution. Useful inorganic sulfate salts include sodium sulfate, potassium sulfate, and/or magnesium sulfate.

This low salt concentration is in contrast to some known solutions containing phosphate salts, which are frequently used for cleansing the small intestine or colon for endoscopy such as sodium phosphate (NaP) (*e.g*., Fleet's Phospho-Soda®). These NaP-containing solutions contain a higher salt concentration than that of the present OSS.

A comparison of one representative OSS solution, SUPREP^{®} (Braintree Laboratories), with a commonly used phosphate solution (Fleet's Phospho-Soda®) is shown in Table 1.

**Table 1**

| **Ion** | **Salt Content (g/15 ml soln)*** | |
|---|---|---|
| | NaP | OSS |
| Na⁺ | 1.668 | 0.48 |
| K⁺ | 0 | 0.117 |
| Mg⁺⁺ | 0 | 0.027 |
| PO₄⁺⁺ | 5.89 | 0 |
| SO₄⁺⁺ | 0 | 1.26 |

| | | |
|---|---|---|
| * Anhydrous weights | | |

As shown in Table 1, each 15 ml of the OSS contains 1.48 g sodium sulfate, 0.27 g potassium sulfate, and 0.135 g magnesium sulfate.

In contrast, each 15 ml of unflavored Fleet's Phospho-Soda® oral saline laxative contains 7.2 g monobasic sodium phosphate monohydrate and 2.7 g dibasic sodium phosphate heptahydrate in a stable, aqueous solution. This NaP solution contains higher levels of salts than the level of salts found in the present OSS, and the level of sulfates in OSS is about 20% of the level of phosphates in NaP. Very high levels of salt may lead to excess salt absorption possibly leading to a potentially dangerous and unnecessary sodium and phosphate load, especially in a patient who has pre-existing electrolyte disturbances or who are prescribed a low-salt diet. More of the salts are absorbed from the NaP solution than from the OSS, in part because of the lower amount of salts present in OSS, but more importantly, because sulfates are more poorly absorbed than are phosphates.

The oral sulfate compositions useful in the methods of the disclosure may alternatively be solid compositions such as tablets, capsules, lozenges, and powder formulations including inorganic sulfate salts such as sodium sulfate, potassium sulfate, and/or magnesium sulfate. The total amount of sulfate in a solid dosage form of the oral sulfate composition is about 0.1 g to about 2.0 g/unit dose, about 0.5 g to about 1.5 g/unit dose, about 0.7 g to about 1.3 g/unit dose, about 0.8 g to about 1.2 g/unit dose, about
0.9 g to about 1.0 g/unit dose, about 0.2 g/unit dose, about 0.3 g/unit dose, about
0.4 g/unit dose, about 0.5 g/unit dose, about 0.6 g/unit dose, about 0.7 g/unit dose, about 0.8 g/unit dose, about 0.9 g/unit dose, about 1.0 g/unit dose, about 1.1 g/unit dose, or about 1.2 g/unit dose.

In one embodiment, the sulfate compositions are administered in a pharmaceutically-acceptable carrier. Any suitable carrier known in the art may be used. Carriers that result in efficient solubilization of the agents are useful. Carriers include, but are not limited to, a solid, liquid, or a mixture of a solid and a liquid. The carriers may take the form of capsules, tablets, pills, powders, lozenges, suspensions, solutions, emulsions, or syrups. The carriers may include substances that act as flavoring agents, lubricants, solubilizers, suspending agents, binders, stabilizers, tablet disintegrating agents, and encapsulating materials. The phrase "pharmaceutically-acceptable" is employed herein to refer to those compounds, materials, compositions, and/or dosage forms which are, within the scope of sound medical judgment, suitable for use in contact with the tissues of patients (human beings and animals) without excessive toxicity, irritation, allergic response, or other problem or complication, commensurate with a reasonable benefit/risk ratio.

Non-limiting examples of materials which can serve as pharmaceutically-acceptable carriers include: (1) sugars, such as lactose, glucose, and sucrose;
(2) starches, such as corn starch and potato starch; (3) cellulose and its derivatives, such as sodium carboxymethyl cellulose, ethyl cellulose, and cellulose acetate;
(4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil, and soybean oil; (10) glycols, such as propylene glycol; (11) polyols, such as glycerin, sorbitol, mannitol, and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline, (18) Ringer's solution, (19) ethyl alcohol; (20) phosphate buffer solutions; and (21) other nontoxic compatible substances employed in pharmaceutical formulations.

The formulations may conveniently be presented in unit dosage form and may be prepared by any methods well known in the art of pharmacy. The amount of sulfate salts which can be combined with a carrier material to produce an oral, single-dosage form will vary depending upon the subject being treated, and the suspected GI condition that the patient is experiencing, among others. The amount of active ingredient that can be combined with a carrier material to produce a single-dosage form is generally be that amount of the compound that produces a therapeutic effect, as described above. Generally, out of 100%, this amount ranges from about 1% to about 99% of active ingredient, from about 5% to about 70%, or from about 10% to about 30%.

Methods of preparing these formulations or compositions include the step of bringing into association a sulfate salt with the carrier and, optionally, one or more accessory ingredients. In general, the formulations are prepared by uniformly and intimately bringing into association sulfate salts with liquid carriers, or finely divided solid carriers, or both, and then, if necessary, shaping the product.

Described herein are solid dosage forms for oral administration (*e.g*., capsules, tablets, pills, lozenges, powders, granules, and the like), the sulfate salts may be mixed with one or more additional ingredients, such as fillers or extenders, such as, but not limited to, starches, lactose, sucrose, glucose, mannitol, and/or silicic acid; binders, such as, but not limited to, carboxymethylcellulose, alginates, gelatin, polyvinyl pyrrolidone, sucrose, and/or acacia; humectants, such as, but not limited to, glycerol; disintegrating agents, such as, but not limited to, agar, calcium carbonate, potato or tapioca starch, alginic acid, certain silicates, and sodium carbonate; solution retarding agents, such as, but not limited to, paraffin; absorption accelerators, such as, but not limited to, quaternary ammonium compounds; wetting agents, such as, but not limited to, cetyl alcohol and glycerol monostearate; absorbents, such as, but not limited to, kaolin and bentonite clay; lubricants, such as, but not limited to, talc, calcium stearate, magnesium stearate, solid polyethylene glycols, sodium lauryl sulfate, and mixtures thereof; and coloring agents. In the case of capsules, tablets, and pills, the pharmaceutical sulfate compositions may also comprise buffering agents. Solid compositions of a similar type may also be employed as fillers in soft and hard-filled gelatin capsules using such excipients as lactose or milk sugars, as well as high molecular weight polyethylene glycols, and the like. A tablet may be made by compression or molding, optionally with one or more accessory ingredients.

In powders, the carrier is a finely-divided solid, which is mixed with an effective amount of a finely-divided sulfate salt. Powders and sprays can contain, in addition to a sulfate salt, excipients, such as lactose, talc, silicic acid, aluminum hydroxide, calcium silicates and polyamide powder, or mixtures of these substances. Spray powders can additionally contain customary propellants, such as chloro-fluorohydrocarbons and volatile unsubstituted hydrocarbons, such as butane and propane.

In oral solutions, suspensions, emulsions or syrups, an effective amount of the sulfate salts may be dissolved or suspended in a carrier, such as sterile water or an organic solvent, such as aqueous propylene glycol. Other compositions can be made by dispersing the agent in an aqueous starch or sodium carboxymethyl cellulose solution or a suitable oil known to the art. The liquid dosage forms may contain inert diluents commonly used in the art, such as, for example, water or other solvents, solubilizing agents and emulsifiers, such as, but not limited to, ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols, and fatty acid esters of sorbitan, and mixtures thereof. Besides inert diluents, the oral compositions can also include adjuvants, such as wetting agents, emulsifying and suspending agents, sweetening, flavoring, coloring, perfuming, and preservative agents.

Suspensions, in addition to the active compound, may contain suspending agents as, for example, ethoxylated isostearyl alcohols, polyoxyethylene sorbitol and sorbitan esters, microcrystalline cellulose, aluminum metahydroxide, bentonite, agar and tragacanth, and mixtures thereof.

### Diagnosis of a GI Pathology

Disclosed is a method of identifying a GI pathology, such as a hemorrhage, tumor, polyp, or lesion in a patient's small bowel and colon. As the oral sulfate composition, according to the disclosure, increases GI transit (or reduces transit time) of a capsule endoscope during the endoscopic procedure, use of this composition provides an improved method of determining the source and cause of a GI pathology in a patient suffering from the same.

Such a patient can be treated as follows. Upon presentation by a patient of symptoms indicative of possible GI pathology, the oral sulfate composition is administered before the capsule endoscope is ingested. Because the full small bowel is traversed in a little more than an hour, the physician can rapidly make the diagnosis of small bowel disease. In the event that the capsule endoscope has a limited battery life, the rapid transit caused by oral sulfate composition reduces the chance of battery failing before the procedure is completed. Likewise, in the event of suspected pathology in the colon, the more rapid delivery of a capsule to and through the colon both speeds up the diagnosis and reduces the risk of a failed procedure due to battery failure. Thus, the oral sulfate composition is of benefit to both upper and lower GI evaluation with capsule endoscopy. Uses of the oral sulfate composition are also envisaged in the event that a patient has unusually slow SI transit to boost transit of the capsule after it is ingested.

Reference will now be made to specific examples illustrating the invention. It is to be understood that the examples are provided to illustrate useful embodiments.

### EXAMPLE

### Transit Time Comparison

To examine whether the present oral sulfate solution has beneficial effects on the small bowel transit rate, this OSS (in the form of Braintree SUPREP®) or the sodium phosphate (NaP) (in the form of Fleet's Phospho-Soda®) solution was administered to healthy volunteers whose demographics (age, gender, weight, and body mass index) are depicted below in Table 2.

**Table 2**

| **Study** | **Age** | **Gender % Male** | **Weight [kg]** | **BMI** |
|---|---|---|---|---|
| OSS (6 + 3oz) N = 23 | 52 ± 8.3 [35 - 68] | 83% | 80 ± 10.5 [59 -98] | 26 ± 3.3 [21 -32] |
| OSP (30 + 25ml) N = 115 | 61 ± 9 [34 - 79] | 61% | 75 ± 13.9 [45 -116] | 26 ± 3.8 [13.7 -36] |

The schedule of representative dosing is shown in Table 3.

**Table 3**

| **PROTOCOL** | | | |
|---|---|---|---|
| **Day** | **Time** | **OSS** | **NaP** |
| 2 | Bedtime | Senna, 4T | Senna, 4 t |
| -1 | All Day | clear liquid diet | clear liquid diet |
| | Evening | 2 L PEG | 2 L PEG |
| 0 Exam Day | Morning | 2 L PEG | 2 L PEG |
| | 0 | capsule ingestion | capsule ingestion |
| | 0 | 6 oz (180 ml OSS (Booster) | 30 ml NaP |
| | + 3 hours | 3 oz (180 ml) OSS (Booster II) | 25 ml NaP |
| | + 5 hours | 10 mg Bisacodyl (suppository | 10 mg Bisacodyl (suppository) |

Under these conditions, the transit times for SB, colon and overall were monitored. In order to monitor the progression of the capsule and/or to verify capsule excretion, the investigator used fluoroscopy or an abdominal x-ray at his/her discretion. Alternatively, the location of the capsule was judged by reviewing the data or images from the data recorder and logging the appropriate anatomical landmarks in the GI tract.

In comparison to the sodium phosphate solution, the present OSS statistically significantly increased transit speed in the small bowel, colon, and the overall transit time, while it did not increase the speed of gastric emptying. The results are shown on Table 4.

**Table 4**

| **Study** | **Gastric Transit (hrs:mins)** | **Colon Transit (hrs:mins)** | **Overall Transit (hrs:mins)** |
|---|---|---|---|
| OSS (6 + 3oz) N=23 | 1:12 ± 1:02 | 1:41 ± 1:37 | 3:47 ± 2:03 |
| NaP (30 + 25ml) N= 115 | 0:45 ± 0:50 | 2:15 ± 1:35 | 4:32 ± 1:49 |

These results demonstrate that the oral sulfate composition according to the disclosure stimulates small bowel and colonic transit. Accordingly, it is useful as a "booster" to propel capsules through the GI tract. It is more potent than NaP in this regard, and the effect occurs at doses of OSS that are previously unknown to have this property.

## Claims

1. Use of an oral sulfate solution to prepare the gastrointestinal tract of a patient for examining the interior of the gastrointestinal tract of the patient via capsule endoscopy,
wherein the oral sulfate solution is to be administered to the patient in an amount effective to purge the gastrointestinal tract of the patient of its contents and to cleanse the gastrointestinal tract, and
wherein the oral sulfate solution is a transit speed enhancer, comprises sodium sulfate, potassium sulfate, and magnesium sulfate, comprises about 0.0096 g/ml to about 0.50 g/ml sulfate in the form of an inorganic sulfate salt, and does not contain a phosphate salt.

2. The use of claim 1, wherein the oral sulfate solution comprises about 0.0095 g/ml to about 0.038 g/ml sodium, about 0.002 g/ml to about 0.009 g/ml potassium, about 0.0005 g/ml to about 0.05 g/ml magnesium, and about 0.02 g/ml to about 0.1 g/ml sulfate.

3. The use of claim 1, wherein the oral sulfate solution comprises about 0.022 g/ml sodium, about 0.005 g/ml potassium, about 0.001 g/ml magnesium, and about 0.07 g/ml sulfate.

4. The use of claim 2 or claim 3, wherein about 15 ml to about 1000 ml of the oral sulfate solution is to be administered.

5. The use of claim 1, wherein the oral sulfate solution is to be administered with a capsule endoscope, or
wherein the oral sulfate solution is to be administered after administration of the capsule endoscope, or
wherein the oral sulfate solution is to be administered more than one time before the administration of the capsule endoscope, or
wherein further an osmotic laxative is also to be administered, and the osmotic laxative is to be administered before the administration of the oral sulfate solution and a capsule endoscope.

6. Use of an oral sulfate solution to prepare the gastrointestinal tract of a patient for determining the source, type, location, and cause of a gastrointestinal pathology in the patient,
wherein the oral sulfate solution is to be administered to the patient experiencing the pathology to prepare the gastrointestinal tract of the patient for capsule colonoscopy,
wherein the oral sulfate solution is to be administered in an amount effective to purge the gastrointestinal tract and to cleanse the gastrointestinal tract, and
wherein the solution comprises magnesium sulfate, potassium sulfate and sodium sulfate, comprises about 0.0096 g/ml to 0.50 g/ml sulfate in the form of an inorganic sulfate salt, and does not contain a phosphate salt.

7. The use of claim 6, wherein the oral sulfate solution comprises about 0.0095 g/ml to about 0.038 g/ml sodium, about 0.002 g/ml to about 0.009 g/ml potassium, about 0.0005 g/ml to about 0.05 g/ml magnesium, and about 0.02 g/ml to about 0.1 g/ml sulfate.

8. The use of claim 7, wherein the oral sulfate solution comprises about 0.022 g/ml sodium, about 0.005 g/ml potassium, about 0.001 g/ml magnesium, and about 0.07 g/ml sulfate.

9. The use of claim 6, wherein 15 ml to 1000 ml of the oral sulfate solution is to be administered.

10. The use of claim 6, wherein the oral sulfate solution is to be administered with the capsule endoscope, or
wherein the oral sulfate solution is to be administered before the administration of the capsule endoscope, or
wherein the oral sulfate solution is to be ingested at more than one time before the administration of the capsule endoscope, or
wherein an osmotic laxative is also to be administered, and the osmotic laxative is to be administered before the administration of the oral sulfate solution and a capsule endoscope.

11. The use of any one of claims 6-10, wherein the gastrointestinal pathology is a hemorrhage, an ulcer, a polyp, a lesion, a precancerous lesion, a cancer, a diverticulitis, or an inflammatory disorder including Crohn's disease, colitis, or ulcerative colitis.

12. The use of claim 1, wherein examining the interior of the gastrointestinal tract of the patient via capsule endoscopy comprises
orally administering an activated capsule endoscope to the patient; positioning a receiver to detect data transmitted from the activated capsule endoscope as it passes through the interior of gastrointestinal tract of the patient; and
analyzing the data detected by the receiver before and/or after the administered capsule endoscope has been expelled from the colon of the patient.

13. The use of claim 6, wherein determining the source, type, location, and cause of the gastrointestinal pathology in the patient comprises orally administering an activated capsule endoscope to the patient;
positioning a receiver to detect and store data transmitted from the capsule endoscope as it passes through the intestinal tract of the patient; and
analyzing the data detected by the receiver before and/or after the administered capsule endoscope has been expelled from the colon of the patient, the data indicating the type, location and cause of the pathology.

14. Use of an oral sulfate solution for preparing the gastrointestinal tract of a patient for capsule endoscopy,
wherein the oral sulfate solution comprises magnesium sulfate, potassium sulfate and sodium sulfate, comprises about 0.0096 g/ml to 0.50 g/ml sulfate in the form of an inorganic sulfate salt, and does not contain a phosphate salt.

15. Use of an oral sulfate solution for enhancing gastrointestinal or small bowel transit speed for capsule endoscopy,
wherein the oral sulfate solution comprises magnesium sulfate, potassium sulfate and sodium sulfate, comprises about 0.0096 g/ml to 0.50 g/ml sulfate in the form of an inorganic sulfate salt, and does not contain a phosphate salt.

## Patentansprüche

1. Verwendung einer oralen Sulfatlösung zur Vorbereitung des Gastrointestinaltraktes eines Patienten zur Untersuchung des Inneren des Gastrointestinaltraktes des Patienten mittels Kapselendoskopie,
wobei die orale Sulfatlösung dem Patienten in einer Menge zu verabreichen ist, die wirksam ist, um den Inhalt des Gastrointestinaltrakts des Patienten zu entleeren und den Gastrointestinaltrakt zu reinigen, und wobei die orale Sulfatlösung als ein Durchgangsgeschwindigkeitsverstärker wirkt, Natriumsulfat, Kaliumsulfat und Magnesiumsulfat enthält, etwa 0,0096 g/ml bis etwa 0,50 g/ml Sulfat in Form eines anorganischen Sulfatsalzes enthält und kein Phosphatsalz enthält.

2. Verwendung nach Anspruch 1, wobei die orale Sulfatlösung etwa 0,0095 g/ml bis etwa 0,038 g/ml Natrium, etwa 0,002 g/ml bis etwa 0,009 g/ml Kalium, etwa 0,0005 g/ml bis etwa 0,05 g/ml Magnesium und etwa 0,02 g/ml bis etwa 0,1 g/ml Sulfat enthält.

3. Verwendung nach Anspruch 1, wobei die orale Sulfatlösung etwa 0,022 g/ml Natrium, etwa 0,005 g/ml Kalium, etwa 0,001 g/ml Magnesium und etwa 0,07 g/ml Sulfat enthält.

4. Verwendung nach Anspruch 2 oder Anspruch 3, wobei etwa 15 ml bis etwa 1000 ml der oralen Sulfatlösung zu verabreichen sind.

5. Verwendung nach Anspruch 1, wobei die orale Sulfatlösung zusammen mit einem Kapselendoskop zu verabreichen ist oder
wobei die orale Sulfatlösung nach Verabreichung des Kapselendoskops zu verabreichen ist oder
wobei die orale Sulfatlösung mehr als einmal vor der Verabreichung des Kapselendoskops zu verabreichen ist oder
wobei ferner ein osmotisches Abführmittel zu verabreichen ist und das osmotische Abführmittel vor der Verabreichung der oralen Sulfatlösung und eines Kapselendoskops zu verabreichen ist.

6. Verwendung einer oralen Sulfatlösung zur Vorbereitung des Gastrointestinaltraktes eines Patienten zur Bestimmung des Ursprungs, des Typs, der Lage und der Ursache einer gastrointestinalen Erkrankung beim Patienten,
wobei die orale Sulfatlösung dem Patienten zu verabreichen ist, der die Erkrankung durchlebt, um den Gastrointestinaltrakt des Patienten für die Kapselkoloskopie vorzubereiten,
wobei die orale Sulfatlösung in einer Menge zu verabreichen ist, die wirksam ist, um den Gastrointestinaltrakt zu entleeren und den Gastrointestinaltrakt zu reinigen, und wobei die Lösung Magnesiumsulfat, Kaliumsulfat und Natriumsulfat enthält, etwa 0,0096 g ml bis 0,50 g ml Sulfat in Form eines anorganischen Sulfatsalzes enthält und kein Phosphatsalz enthält.

7. Verwendung nach Anspruch 6, wobei die orale Sulfadösung etwa 0,0095 g/ml bis etwa 0,038 g/ml Natrium, etwa 0,002 g/ml bis etwa 0,009 g/ml Kalium, etwa 0,0005 g/ml bis etwa 0,05 g/ml Magnesium und etwa 0,02 g/ml bis etwa 0,1 g/ml Sulfat enthält.

8. Verwendung nach Anspruch 7, wobei die orale Sulfatlösung etwa 0,022 g/ml Natrium, etwa 0,005 g/ml Kalium, etwa 0,001 g/ml Magnesium und etwa 0,07 g/ml Sulfat enthält.

9. Verwendung nach Anspruch 6, wobei 15 ml bis 1000 ml der oralen Sulfatlösung zu verabreichen sind.

10. Verwendung nach Anspruch 6, wobei die orale Sulfatlösung zusammen mit dem Kapselendoskop zu verabreichen ist oder
wobei die orale Sulfatlösung vor der Verabreichung des Kapselendoskops zu verabreichen ist oder
wobei die orale Sulfatlösung mehr als einmal vor der Verabreichung des Kapselendoskops aufzunehmen ist oder
wobei auch ein osmotisches Abführmittel zu verabreichen ist und das osmotische
Abführmittel vor der Verabreichung der oralen Sulfatlösung und eines Kapselendoskops zu verabreichen ist.

11. Verwendung nach einem der Ansprüche 6-10, wobei die gastrointestinale Erkrankung eine Blutung, ein Geschwür, ein Polyp, eine Läsion, eine präkanzeröse Läsion, ein Krebs, eine Divertikulitis oder eine entzündliche Erkrankung einschließlich Morbus Crohn, Colitis oder Colitis ulcerosa ist.

12. Verwendung nach Anspruch 1, wobei die Untersuchung des Inneren des Gastrointestinaltraktes des Patienten mittels einer Kapselendoskopie umfasst orales Verabreichen eines aktivierten Kapselendoskops an den Patienten; Positionieren eines Empfängers zum Erfassen von Daten, die von dem aktivierten Kapselendoskop übertragen werden, wenn es das Innere des Gastrointestinaltraktes des Patienten durchläuft; und
Analysieren der vom Empfänger erfassten Daten vor und/oder nachdem das verabreichte Kapselendoskop aus dem Dickdarm des Patienten ausgestoßen worden ist.

13. Verwendung nach Anspruch 6, wobei das Bestimmen des Ursprungs, des Typs, der Lage und der Ursache der gastrointestinalen Erkrankung in dem Patienten umfasst orales Verabreichen eines aktivierten Kapselendoskops an den Patienten; Positionieren eines Empfängers zum Erfassen und Speichern von Daten, die von dem Kapselendoskop übertragen werden, wenn es den Intestinaltrakt des Patienten durchläuft; und
Analysieren der vom Empfänger erfassten Daten vor und/oder nachdem das verabreichte Kapselendoskop aus dem Dickdarm des Patienten ausgestoßen worden ist,
wobei die Daten den Typ, den Ort und die Ursache der Erkrankung angeben.

14. Verwendung einer oralen Sulfatlösung zur Vorbereitung des Gastrointestinaltraktes eines Patienten für die Kapselendoskopie,
wobei die orale Sulfatlösung Magnesiumsulfat, Kaliumsulfat und Natriumsulfat enthält, etwa 0,0096 g/ml bis 0,50 g/ml Sulfat in Form eines anorganischen Sulfatsalzes enthält und kein Phosphatsalz enthält.

15. Verwendung einer oralen Sulfatlösung zur Verbesserung der gastrointestinalen oder Dünndarm-Durchgangsgeschwindigkeit für die Kapselendoskopie,
wobei die orale Sulfatlösung Magnesiumsulfat, Kaliumsulfat und Natriumsulfat enthält, etwa 0,0096 g/ml bis 0,50 g/ml Sulfat in Form eines anorganischen Sulfatsalzes enthält und kein Phosphatsalz enthält.

## Revendications

1. Utilisation d'une solution de sulfate orale pour préparer le tractus gastro-intestinal d'un patient pour examiner l'intérieur du tractus gastro-intestinal du patient par endoscopie par capsule,
dans laquelle la solution de sulfate orale doit être administrée au patient en une quantité efficace pour purger le tractus gastro-intestinal du patient de son contenu et pour nettoyer le tractus gastro-intestinal, et
dans laquelle la solution de sulfate orale est un agent d'amélioration de la vitesse de transit, comprend du sulfate de sodium, du sulfate de potassium et du sulfate de magnésium, comprend d'environ 0,0096 g/ml à environ 0,50 g/ml de sulfate sous la forme d'un sel de sulfate inorganique, et ne contient pas de sel de phosphate.

2. Utilisation selon la revendication 1, dans laquelle la solution de sulfate orale comprend d'environ 0,0095 g/ml à environ 0,038 g/ml de sodium, d'environ 0,002 g/ml à environ 0,009 g/ml de potassium, d'environ 0,0005 g/ml à environ 0,05 g/ml de magnésium et d'environ 0,02 g/ml à environ 0,1 g/ml de sulfate.

3. Utilisation selon la revendication 1, dans laquelle la solution de sulfate orale comprend environ 0,022 g/ml de sodium, environ 0,005 g/ml de potassium, environ 0,001 g/ml de magnésium, et environ 0,07 g/ml de sulfate.

4. Utilisation selon la revendication 2 ou la revendication 3, dans laquelle d'environ 15 ml à environ 1000 ml de la solution de sulfate orale doivent être administrés.

5. Utilisation selon la revendication 1, dans laquelle la solution de sulfate orale doit être administrée avec une vidéocapsule, ou
dans laquelle la solution de sulfate orale doit être administrée après l'administration de la vidéocapsule, ou
dans laquelle la solution de sulfate orale doit être administrée plus d'une fois avant l'administration de la vidéocapsule, ou
dans laquelle en outre un laxatif osmotique doit également être administré, et le laxatif osmotique doit être administré avant l'administration de la solution de sulfate orale et d'une vidéocapsule.

6. Utilisation d'une solution de sulfate orale pour préparer le tractus gastro-intestinal d'un patient pour déterminer la source, le type, l'emplacement et la cause d'une pathologie gastro-intestinale chez le patient,
dans laquelle la solution de sulfate orale doit être administrée au patient souffrant de la pathologie pour préparer le tractus gastro-intestinal du patient pour coloscopie par capsule,
dans laquelle la solution de sulfate orale doit être administrée en une quantité efficace pour purger le tractus gastro-intestinal et pour nettoyer le tractus gastro-intestinal, et
dans laquelle la solution comprend du sulfate de magnésium, du sulfate de potassium et du sulfate de sodium, comprend d'environ 0,0096 g/ml à 0,50 g/ml de sulfate sous la forme d'un sel de sulfate inorganique, et ne contient pas de sel de phosphate.

7. Utilisation selon la revendication 6, dans laquelle la solution de sulfate orale comprend d'environ 0,0095 g/ml à environ 0,038 g/ml de sodium, d'environ 0,002 g/ml à environ 0,009 g/ml de potassium, d'environ 0,0005 g/ml à environ 0,05 g/ml de magnésium et d'environ 0,02 g/ml à environ 0,1 g/ml de sulfate.

8. Utilisation selon la revendication 7, dans laquelle la solution de sulfate orale comprend environ 0,022 g/ml de sodium, environ 0,005 g/ml de potassium, environ 0,001 g/ml de magnésium, et environ 0,07 g/ml de sulfate.

9. Utilisation selon la revendication 6, dans laquelle 15 ml à 1000 ml de la solution de sulfate orale doivent être administrés.

10. Utilisation selon la revendication 6, dans laquelle la solution de sulfate orale doit être administrée avec la vidéocapsule, ou
dans laquelle la solution de sulfate orale doit être administrée avant l'administration de la vidéocapsule, ou
dans laquelle la solution de sulfate orale doit être ingérée plus d'une fois avant l'administration de la vidéocapsule, ou
dans laquelle un laxatif osmotique doit également être administré, et le laxatif osmotique doit être administré avant l'administration de la solution de sulfate orale et d'une vidéocapsule.

11. Utilisation selon l'une quelconque des revendications 6-10, dans laquelle la pathologie gastro-intestinale est une hémorragie, un ulcère, un polype, une lésion, une lésion précancéreuse, un cancer, une diverticulite, ou un trouble inflammatoire incluant la maladie de Crohn, la colite ou la colite ulcéreuse.

12. Utilisation selon la revendication 1, dans laquelle l'examen de l'intérieur du tractus gastro-intestinal du patient par le biais d'une endoscopie par capsule comprend
l'administration orale d'une vidéocapsule activée au patient ; le positionnement d'un récepteur pour détecter des données transmises depuis la vidéocapsule activée quand il traverse l'intérieur du tractus gastro-intestinal du patient ; et l'analyse des données détectées par le récepteur avant et/ou après que la vidéocapsule administré a été expulsé du colon du patient.

13. Utilisation selon la revendication 6, dans laquelle la détermination de la source, du type, de l'emplacement et de la cause de la pathologie gastro-intestinale chez le patient comprend l'administration orale d'une vidéocapsule activée au patient ;
le positionnement d'un récepteur pour détecter et stocker des données transmises depuis la vidéocapsule quand il traverse le tractus gastro-intestinal du patient ; et
l'analyse des données détectées par le récepteur avant et/ou après que la vidéocapsule administré a été expulsé du colon du patient,
les données indiquant le type, l'emplacement et la cause de la pathologie.

14. Utilisation d'une solution de sulfate orale pour préparer le tractus gastro-intestinal d'un patient pour endoscopie par capsule,
dans laquelle la solution de sulfate orale comprend du sulfate de magnésium, du sulfate de potassium et du sulfate de sodium, comprend d'environ 0,0096 g/ml à 0,50 g/ml de sulfate sous la forme d'un sel de sulfate inorganique, et ne contient pas de sel de phosphate.

15. Utilisation d'une solution de sulfate orale pour améliorer la vitesse de transit gastro-intestinal ou de l'intestin grêle pour endoscopie par capsule,
dans laquelle la solution de sulfate orale comprend du sulfate de magnésium, du sulfate de potassium et du sulfate de sodium, comprend d'environ 0,0096 g/ml à 0,50 g/ml de sulfate sous la forme d'un sel de sulfate inorganique, et ne contient pas de sel de phosphate.
